(19) Europäisches Patentamt
European Patent Office
Office européen des brevets

(11) Publication number: 0 045 827 B1

(12) # EUROPEAN PATENT SPECIFICATION

(45) Date of publication of patent specification: 02.11.83

(51) Int. Cl.[3]: C 10 M 1/32, C 10 M 1/38

(21) Application number: 80302764.8

(22) Date of filing: 12.08.80

(54) **Lubricant compositions containing antioxidant mixtures of triazoles and thiadiazoles**

(30) Priority: 00.00.00

(43) Date of publication of application: 17.02.82 Bulletin 82/07

(45) Publication of the grant of the patent: 02.11.83 Bulletin 83/44

(84) Designated Contracting States: BE DE FR GB IT NL SE

(56) References cited:
EP - A - 0 006 710
GB - A - 1 520 743
US - A - 2 719 125

(73) Proprietor: MOBIL OIL CORPORATION
150 East 42nd Street
New York New York 10017 (US)

(72) Inventor: Shim, Joosup
6 South Marion Avenue
Wenonah New Jersey (US)

(74) Representative: West, Alan Harry
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 045 827 B1

# Lubricant compositions containing antioxidant mixtures of triazoles and thiadiazoles

The invention relates to lubricant compositions which are normally susceptible to oxidative deterioration.

Triazoles have been employed in lubricant compositions as metal deactivators. For example U.S. Patent No. 3,597,353 discloses the use of tetrahydrobenzotriazole as a metal deactivating additive for natural and synthetic lubricants. Similarly, U.S. Patent No. 3,413,227 describes the use of $C_2$—$C_{20}$ alkyl-substituted benzotriazole as corrosion or tarnish inhibitors. U.S. Patent No. 4,060,491 discloses the use of 5-alkyl benzotriazoles, for reducing wear between moving steel-on-steel surfaces.

Reaction products of benzotriazoles are also known. For example, U.S. Patent No. 3,788,993 describes the reaction of benzotriazoles with alkyl or alkenyl succinic anhydrides to form products which impart corrosion inhibiting properties to lubricating oils.

U.S. Patent No. 4,048,082, discloses the use of esters of adducts of benzotriazole and unsaturated dicarboxylic acids or anhydrides for imparting antirust properties to organic compositions.

We now found that combinations of benzotriazole adducts with certain thiadiazoles have extremely valuable properties. In particular, they confer good antioxidation and corrosion-resistant properties on lubricating oils.

In accordance with the present invention, we therefore provide a lubricating composition containing a major amount of a lubricant and an antioxidant amount of a mixture of (1) an adduct of a benzotriazole compound and a vinyl ether or a vinyl ester of a carboxylic acid and (2) an alkyl dimercapto thiadiazole.

The benzotriazole adducts used in the mixtures of the present invention are formed by reacting a benzotriazole compound with a vinyl ether or a vinyl ester of a carboxylic acid. They are described in our pending European Patent Application No. 79301128 (Publn. No. 6710).

The benzotriazole compounds which may be used to form the adducts have the formula:

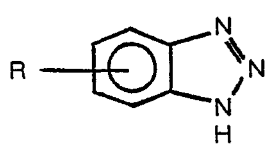

where R is hydrogen or hydrocarbyl containing from 1 to 12 carbon atoms, and preferably is hydrogen or an alkyl group containing from 1 to 8 carbon atoms. Particularly preferred are benzotriazole and tolutriazole.

The vinyl ethers and vinyl esters which may be utilized in forming the adducts of the present invention have the formulae:

$$R'CH{=}CHOR'' \text{ and } R'CH{=}CHO\overset{\overset{\displaystyle O}{\|}}{C}{-}R'''$$

respectively, where R′ is hydrogen or an alkyl group containing from 1 to 8 carbon atoms in any isomeric arrangement, R″ is an alkyl group containing from 1 to 18 carbon atoms in any isomeric arrangement, and R‴ may be the same as R″ or may be an aryl group, an alkaryl group or an aralkyl group containing from 1 to 18 carbon atoms.

Preferred are those vinyl ethers and vinyl esters wherein R′ is hydrogen or an alkyl group containing from 1 to 11 carbon atoms, R″ is an alkyl group containing from 1 to 8 carbon atoms, and R‴ is an alkyl, aralkyl or alkaryl group containing from about 1 to 14 carbon atoms.

Particularly preferred are those vinyl ethers and vinyl esters wherein R′ is hydrogen or alkyl of 1 to 2 carbon atoms, R″ is an alkyl group containing from 1 to 4 carbon atoms, and R‴ is an alkyl or aryl group containing from 1 to 6 carbon atoms.

The benzotriazole adducts are formed by reacting the benzotriazole compound with the vinyl ether or vinyl ester of a carboxylic acid in proportions, expressed as molar ratios of benzotriazole compound to vinyl ether or vinyl carboxylate, from 1:1 to 1:10, with from 1:1 to 1:1.5 being preferred.

Temperatures from 25°C to 150°C, preferably from 80°C to 120°C are used. In general, the reactants are contacted for 1 to 8 hours, preferably 2 to 4 hours. The particular reaction times used will depend on the temperature and the reactants employed and at higher temperatures, the reaction time may be shorter than the time at lower temperatures, for a given pair of reactants.

The reaction often proceeds without the presence of any catalyst. However, catalysts of an acidic nature, such as acetic acid, propionic acid, toluenesulfonic acid, phosphonic or polyphosphonic and methanesulfonic acids may be employed. Basic catalysts can also be used. Typical examples include sodium or potassium alkoxides, sodium or potassium metal and their hydroxides.

The benzotriazole products of the present invention may comprise several isomers, i.e., the vinyl ethers and vinyl esters may connect to the benzotriazole in either the 1-H or 2-H position. Also, both

Markownikow and anti-Markownikow additions may occur. It has been found that each isomer is individually effective in imparting the improved antioxidant and anti-corrosion properties to the lubricant compositions. Accordingly, as used herein the term "adducts" or "benzotriazole product(s)" may refer to any of the isomers produced, or the mixture of isomers.

The thiadiazoles used in the compositions are the alkyl dimercapto thiadiazoles. The preferred thiadiazoles are the 2,5-dimercapto-1,3,4-thiadiazoles of the formula:

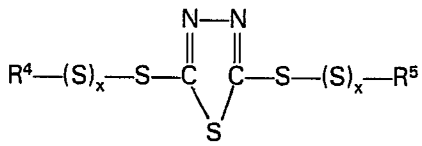

where $R^4$ and $R^5$ are hydrocarbyl groups, either the same or different, containing from 1 to 30 carbon atoms and x is 0 to 8. $R^4$ and $R^5$ can be, for example, alkyl, aryl, alkaryl or aralkyl, preferably alkyl, and specific examples of which are methyl, butyl, octyl, decyl, dodecyl, octadecyl, phenyl, tolyl, benzyl, and the like. They can be made in accordance with the method described in U.S. 2,719,125 and are commercially available.

The present invention provides improved resistance to oxidation of lubricating media including liquid oils of lubricating viscosity or greases. Both mineral (paraffinic or naphthenic) and synthetic oils as well as mixtures of them may be employed as the lubricant or the grease vehicle. A wide variety of materials may be employed as thickening or gelling agents for greases. These may include the conventional metal salts or soaps and other thickening agents such as the non-soap thickeners, for example, the surface-modified clays and silicas, aryl ureas and calcium complexes.

Typical synthetic vehicles include polyolefins, polyglycols and esters such as the polybutenes, hydrogenated polydecenes, polypropylene glycol, polyethylene glycol, trimethylol propane esters, neopentyl and pentaerythritol esters, di(2-ethylhexyl) sebacate and di(2-ethylhexyl) adipate.

The antioxidant mixtures of the present invention are unexpectedly effective in mineral oils with a high sulfur content.

The lubricants can also contain other materials, for example, corrosion inhibitors, extreme pressure agents, viscosity index improvers, co-antioxidants and anti-wear agents.

The mixtures of the present invention may be employed in any amount which is effective for imparting the desired degree of oxidation improvement. The mixture is effectively employed in amounts from 0.001% to 0.2% by weight, and preferably from 0.01% to 0.1% of the total weight of the composition. When preparing the mixture of the two compounds, from 20% to 80%, preferably 50% to 70% of the benzotriazole adduct and from 80% to 20%, preferably from 30% to 50% of the thiadiazole are used. All percentages are by weight of total mixture.

The following examples will provide specific illustrations of the invention described hereinabove.

## Example 1

Adducts of Benzotriazole and n-Butyl Vinyl Ether

A mixture of 59.5 g of benzotriazole, 100 g. of *n*-butyl vinyl ether and 100 ml. of benzene was heated at 90°C (refluxing) for 6 hr. An additional 50 g. of *n*-butyl vinyl ether was added and refluxing at 90°C was continued for about 5 hours after which unreacted *n*-butyl vinyl ether and the benzene solvent were removed by distillation. To the residue, petroleum ether (bp 30—60°C) was added and precipitated unreacted benzotriazole (9.1 g.) was removed by filtering. Distillation of solvent from the filtrate left 90 g. (97%) of the mixed isomeric addition product.

Gas chromatography showed that the reaction product consisted of two major components (isomers) which could not be separated by distillation. A narrow fraction, having a bp of 100—103°C at less than 13.3 Pa (0.1 mm. Hg.) was estimated, from gas chromatography, to be a 30:70 mixture of isomeric mono-addition products.

Elemental analysis conformed to a mono-adduct reaction product having the empirical formula $C_{12}H_{17}N_3O$:

| Analysis (wt.) | C | H | N |
|---|---|---|---|
| Calculated for $C_{12}H_{17}N_3O$: | 65.73 | 7.81 | 19.16 |
| Found: | 66.34 | 7.63 | 19.9 |

One isomer (designated Isomer A) was separated by elution from a column packed with Alcoa (F—20 alumina (Alcoa is a trade mark), using petroleum ether (bp 30—60°C) as a solvent. A satisfactory elemental analysis for the mono-adduct was obtained:

| Analysis for Isomer A (wt.%) | C | H | N |
|---|---|---|---|
| Calculated for $C_{12}H_{17}N_3O$: | 65.73 | 7.81 | 19.16 |
| Found: | 66.31 | 8.08 | 19.0 |

The ultra-violet spectrum of Isomer A showed maxima at 284.2, 277.6, 272.5 and a shoulder of 266.0 nm. The ultra-violet spectrum for the starting benzotriazole was significantly different, having maxima at 276, 259 and 254 nm. The nmr proton spectrum had a quartet, indicating splitting of the proton by an adjacent methyl group showing that the isomer is a Markownikow adduct. Based on these data, it is concluded that Isomer A has the following structure:

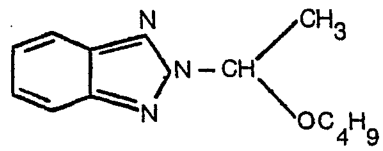

The second isomer (designated isomer B) was isolated by benzene elution from a neutral alumina column. A satisfactory elemental analysis for the mono adduct was also obtained:

| Analysis for Isomer B (wt.%) | C | H | N |
|---|---|---|---|
| Calculated for $C_{12}H_{12}N_3O$: | 65.73 | 7.81 | 19.16 |
| Found: | 65.60 | 7.78 | 19.4 |

The ultra-violet spectrum of Isomer B showed maxima at 282, 261, and 254.5 nm. This is similar to the ultra-violet spectrum of the starting benzotriazole which showed maxima at 276, 259, and 254 nm. The infrared spectrum of Isomer B showed significant differences from that of Isomer A. The nmr proton spectrum of lomer B also had a quartet, indicating that a methyl group is splitting a single adjacent proton and this isomer is also a Markownikowadduct. Based on these data, it is concluded that Isomer B has the following structure:

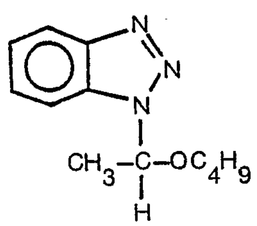

Example 2

Tolutriazole and n-Butyl Vinyl Ether Addition Product

A mixture of 190 g. of tolutriazole (5-methylbenzotriazole), 300 g. of *n*-butyl vinyl ether and 200 ml. of benzene was heated, while refluxing at 88—92°C, for a total of 14 hrs. Unreacted *n*-butyl vinyl ether and benzene solvent were removed by distillation under reduced pressure and the residue was cooled and filtered through a bed of Super Cel (trade mark) filter media. There was obtained 306 g. of the addition product, a clean dark amber liquid representing a yield of 92%.

Example 3

Benzotriazole and Vinyl Acetate Addition Product — Base Catalyzed

To benzotriazole (59.5 g.) and potassium *tert*-butoxide in toluene (100 ml.), heated at 103—115°C, vinyl acetate (86 g.) was added during about 2.25 hr. The reaction mixture was then heated at 98°C while stirring for an additional 5 hr. period. The reaction mixture was washed with water, dried and stripped of solvent by rotary evaporation. The addition product (50.5 g.) m.p. 63—64°C was obtained from the residue by extraction with cyclohexane. Recrystallization from benzene gave a white crystalline solid, m.p. 64—65°C. The infrared spectrum was consistent with the adduct structure. Elemental and analysis was satisfactory:

| | C | H | N |
|---|---|---|---|
| Analysis calculated for $C_{10}H_{11}O_2N_3$: | 58.53 | 5.40 | 20.48 |
| Found: | 58.81 | 5.46 | 20.4 |

Example 4

Evaluation of Products

The alkyl dimercapto thiadiazole used in the blends described below was purchased as Amoco 150. It is the product of Example VI of U.S. Patent No. 3,719,125.

Three different blends, made up as follows, were evaluated in various tests:

1. A solvent paraffinic neutral mineral oil (viscosity: $3.5 \times 10^{-5}$ $m^2/s$ 35 cSt at 38°C) with 0.55% of a standard additive system and comprising (1) 60% of the reaction product of 1 mole of butyl vinyl ether and 1 mole of tolytriazole and (2) 40% of the alkyl dimercapto thiadiazole.

2. A blend containing highly refined base stock (viscosity: $3.5 \times 10^{-5}$ $m^2/s$ (35 cSt) at 38°C; viscosity index of 111) blended with a standard additive system.

3. A blend containing 25% of 1 and 75% of 2.

These tests included determination of neutralization number (NN) foaming tendency, rusting, copper corrosion and rotary bomb oxidation test (RBOT).

Copper Corrosion Test — ASTM D—130

A polished copper strip is immersed in the sample and heated at a temperature and for a time characteristic of the material being tested. At the end of this period the copper strip is removed, washed, and compared with the ASTM Copper Strip Corrosion Standards. A temperature of 49°C (120°F) and a time of 3 hours were used.

Rotary Bomb Oxidation Test (RBOT) — ASTM D—2272

The test oil, water, and copper catalyst coil, contained in a covered glass container, are placed in a bomb equipped with a pressure gage. The bomb is charged with oxygen to a pressure of 620 kPa., placed in a constant-temperature oil bath set at 150°C, and rotated axially at 100 rpm at an angle of 30° from the horizontal. The time for the test oil to react with a given volume of oxygen is measured, completion of the time being indicated by a specific drop in pressure, here a pressure drop of 172 kPa.

Rust Test — ASTM D—665

The method involves stirring a mixture of 300 ml. of the oil under test with 30 ml. of distilled or synthetic sea water, as required, at a temperature of 60°C with a cylindrical steel specimen completely immersed therein. It is customary to run the test for 24 hours; however, the test period may, at the discretion of the contracting parties, be for a shorter or longer period. The test was run for 24 hours using synthetic sea water at 60°C.

Foam Test — ASTM D—892

The sample, maintained at a temperature of 24°C is blown with air at a constant rate for 5 minutes, then allowed to settle for 10 minutes. The volume of foam is measured at the end of both periods. The test is repeated on a second sample at 93°C and then, after collapsing the foam, at 24°C.

The results are summarized in Table 1.

TABLE 1

| Blend | 1 | 2 | 3 |
|---|---|---|---|
| NN | 0.07 | 0.20 | 0.32 |
| Foam Test | 180/0 | 225/0 | 190/0 |
| Rust Test | Pass | Pass | Pass |
| Copper Corrosion Test | 1B | 1A | 1A |
| RBOT, Min. | 390/425 | 260 | 110 |

Other blends were made up as follows:

1. A base fluid containing 99.45% of a $3.5 \times 10^{-5}$ $m^2/s$ (35 cSt) (38°C) turbine base oil and a standard additive package.

2. Blend 1 plus 0.05% by weight of a mixture containing (1) 60% by weight of the reaction product of 1 mole of butyl vinyl ether and 1 mole of tolyltriazole and (2) 40% by weight of the alkyl dimercapto thiadiazole,

3. Blend 1 plus 0.05% by weight of the reaction product (1) of Blend 2,

4. Blend 1 plus 0.05% by weight of the alkyl dimercapto thiadiazole of Blend 2.

These were evaluated in the tests described below.

Total Oxidation Products Test (CIGRE) — IP 280

Oxygen is passed for 164 hours through a sample of the oil with the added soluble metal catalyst, iron and copper and maintained at 120°C. The volatile acid products, the acidity of the oil and the sludge formed are determined. If the measurement of the length of time to obtain a pronounced change

in the rate of evolution of volatile acid is required, an acidity/time curve can be obtained by daily determinations of the volatile acids. The catalyst is a soluble copper salt.

Rotary Bomb Oxidation Test (RBOT) — ASTM D—2272
Described above.

Turbine Oil Stability Test (TOST) — ASTM D—943
The oil sample is subjected to a temperature of 90°C in the presence of water, oxygen and an iron-copper catalyst. The test was carried out for 2500 hours.

The results were as follows:

TABLE 2

| Blend | 1 | 2 | 3 | 4 |
|---|---|---|---|---|
| CIGRE, Total oxidation Products | 5.53 | 0.29 | 1.15 | 5.22 |
| RBOT Min. | 315 | 440 | 400 | 395 |
| TOST % Sludge, by wt. | 0.76 | 0.16 | 0.28 | 0.36 |

**Claims**

1. A lubricating composition comprising a major proportion of a lubricant and an antioxidant amount of a mixture of (1) an adduct of a benzotriazole compound and a vinyl ether or a vinyl ester of a carboxylic acid and (2) an alkyl dimercapto thiadiazole.

2. A composition of Claim 1 in which the benzotryazole used to form the adducts has the formula

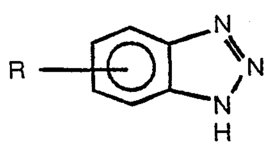

wherein R is hydrogen or hydrocarbyl containing from 1 to 12 carbon atoms.

3. A composition of Claim 2 in which R is alkyl group containing 1 to 8 carbon atoms.

4. A composition of Claim 3 in which R is tertiary octyl.

5. A composition of any of Claims 1 to 4 in which the vinyl ether has the formula

$$R'CH{=}CHOR''$$

where R′ is hydrogen or an isomeric alkyl group containing from 1 to 8 and R″ is an isomeric alkyl group having 1 to 18 carbon atoms.

6. A composition of Claim 5 wherein the vinyl ether is n-butyl vinyl ether.

7. A composition of any of Claims 1 to 4 in which the vinyl ester has the formula

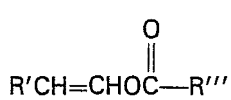

where R′ is hydrogen or an isomeric group containing from 1 to 8 carbon atoms and R‴ is an isomeric alkyl group containing from 1 to 18 carbon atoms or is an aryl group, an alkaryl group or an aralkyl group containing frofm 1 to 18 carbon atoms.

8. A composition of Claim 7 in which the vinyl ester is vinyl acetate.

9. A composition of Claim 1 in which the dimercapto thiadiazole has the formula

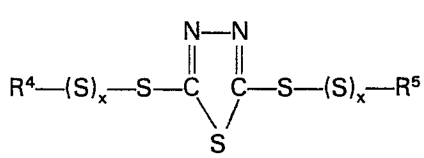

wherein $R^4$ and $R^5$ are hydrocarbyl groups containing from 1 to 30 carbon atoms and x is 0 to 8.

**Revendications**

1. Composition lubrifiante, caractérisée en ce qu'elle comprend une proportion principale d'un lubrifiant et une quantité antioxydante d'un mélange (1) d'un produit d'addition d'un composé du benzotriazole et d'un éther ou d'un ester vinylique d'un acide carboxylique et (2) d'un alkyl-dimercapto-thiadiazole.

2. Composition selon la revendication 1, caractérisée en ce que le benzotriazole, utilisé pour former les produits d'addition, a la formule:

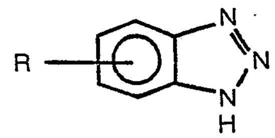

dans laquelle R est un atome d'hydrogène ou un groupe hydrocarboné de 1 à 12 atomes de carbone.

3. Composition selon la revendication 2, caractérisée en ce que R est un groupe alkyle de 1 à 8 atomes de carbone.

4. Composition selon la revendication 3, caractérisée en ce que R est un groupe octyle tertiaire.

5. Composition selon l'une quelconque des revendications 1 à 4, caractérisée en ce que l'éther vinylique a la formule:

$$R'CH{=}CHOR''$$

dans laquelle R' représente un atome d'hydrogne ou un groupe alkyle isomère de 1 à 8 atomes de carbone et R'' représente un groupe alkyle isomère de 1 à 18 atomes de carbone.

6. Composition selon la revendication 5, caractérisée en ce que l'éther vinylique est le n-butyl vinyléther.

7. Composition selon l'une quelconque des revendications 1 à 4, caractérisée en ce que l'ester vinylique a la formule:

$$R'CH{=}CHO\overset{\overset{\displaystyle O}{\|}}{C}{-}R''$$

dans laquelle R' est un atome d'hydrogène ou un groupe alkyle isomère contenant entre 1 et 8 atomes de carbone et R'' est un groupe alkyle isomère contenant entre 1 et 18 atomes de carbone ou un groupe aryle, alkaryle ou aralkyle contenant entre 1 et 18 atomes de carbone.

8. Composition selon la revendication 7, caractérisée en ce que l'ester vinylique est l'acétate de vinyle.

9. Composition selon la revendication 1, caractérisée en ce que le dimercapto thiadiazole a la formule

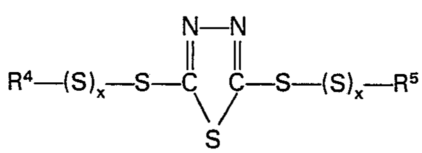

dans laquelle $R^4$ et $R^5$ représentent des groupes hydrocarbonés contenant entre 1 et 30 atomes de carbone et x a une valeur comprise entre 0 et 8.

**Patentansprüche**

1. Schmierstoff-Zusammensetzung, die einen Hauptanteil eines Schmierstoffs und eine Menge eines Antioxidans enthält, das aus einer Mischung von (1) einem Addukt von einer Benzotriazol-Verbindung und einem Vinylether oder Vinylester einer Carbonsäure und (2) einem Alkyldimercaptothiadiazol besteht.

2. Zusammensetzung nach Anspruch 1, bei der das zur Bildung der Addukte verwendete Benzotriazol die folgende Formel aufweist:

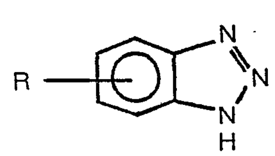

in der R ein Wasserstoffatom oder ein Kohlenwasserstoffrest mit 1 bis 12 C-Atomen ist.

3. Zusammensetzung nach Anspruch 2, in der R eine Alkylgruppe mit 1 bis 8 Kohlenstoffatomen ist.

4. Zusammensetzung nach Anspruch 3, in der R eine tertiäre Octylgruppe ist.

5. Zusammensetzung nach einem der Ansprüche 1 bis 4, bei der der Vinylether die folgende Formel aufweist:

$$R'CH{=}CHOR'',$$

in der R' ein Wasserstoffatom oder eine isomere Alkylgruppe, die von 1 bis 8 Kohlenstoffatome enthält, und R'' eine isomere Alkylgruppe, die 1 bis 18 Kohlenstoffatome enthält, sind.

6. Zusammensetzung nach Anspruch 5, in der der Vinylether n-Butylvinylether ist.

7. Zusammensetzung nach einem der Ansprüche 1 bis 4, bei der der Vinylester die folgende Formel aufweist:

$$R'CH{=}CHOC(O){-}R'''$$

in der R' Wasserstoff oder eine isomere Gruppe die von 1 bis 8 Kohlenstoffatome enthält, und R''' eine isomere Alkylgruppe, die von 1 bis 18 Kohlenstoffatome enthält oder eine Arylgruppe, eine Alkaryl-Gruppe oder eine Aralkyl-Gruppe mit von 1 bis 18 Kohlenstoffatomen, sind.

8. Zusammensetzung nach Anspruch 7, in der der Vinylester Vinylacetat ist.

9. Eine Zusammensetzung nach Anspruch 1, in der das Dimercaptothiadiazol die folgende Formel aufweist:

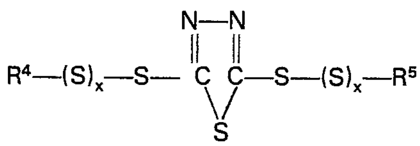

in der $R^4$ und $R^5$ Kohlenwasserstoff-Gruppen sind, die von 1 bis 30 Kohlenstoffatome enthalten, und x 0 bis 8 ist.